(19) Europäisches Patentamt

European Patent Office

Office européen des brevets

(11) Veröffentlichungsnummer: **0 246 532 B1**

(12) **EUROPÄISCHE PATENTSCHRIFT**

(45) Veröffentlichungstag der Patentschrift: **01.07.92**

(21) Anmeldenummer: **87106831.8**

(22) Anmeldetag: **12.05.87**

(51) Int. Cl.⁵: **A61K 31/71**, //(A61K31/71, 31:44),(A61K31/71,31:17), (A61K31/71,31:505)

(54) **Coccidiozide Mittel.**

(30) Priorität: **14.05.86 DE 3616279**

(43) Veröffentlichungstag der Anmeldung:
**25.11.87 Patentblatt 87/48**

(45) Bekanntmachung des Hinweises auf die Patenterteilung:
**01.07.92 Patentblatt 92/27**

(84) Benannte Vertragsstaaten:
**AT CH DE ES FR GB GR IT LI LU NL SE**

(56) Entgegenhaltungen:
**EP-A- 0 015 110**
**EP-A- 0 076 059**

(73) Patentinhaber: **HOECHST AKTIENGESELL-SCHAFT**
**Postfach 80 03 20**
**W-6230 Frankfurt am Main 80(DE)**

(72) Erfinder: **Raether, Wolfgang, Dr.**
**Falkensteinstrasse 6**
**W-6072 Dreieich(DE)**

**Beschreibung**

Salinomycin und Narasin (= 4-Methylsalinomycin) sind als Polyetherantibioticum aus J. Antibiotics 30, S. 530 - 532 (1977) bekannt, s. auch Merck Index 10. Aufl., S. 920 (1983). Dort wird auch deren Verwendung als Anticoccidiosemittel beschrieben. Diese Verbindungen werden durch Fermentation hergestellt, s. DE-PS 2,525,095.

Es wurde nun gefunden, daß die Wirkung von Salinomycin und Narasin bei deren Kombination mit anderen bekannten coccidioziden Wirkstoffen über das zu erwartende Maß hinaus erhöht wird.

Gegenstand der Erfindung sind daher coccidiozide Mittel, die gekennzeichnet sind durch einen Gehalt an einem Polyetherantibiotikum aus der Gruppe Salinomycin oder Narasin oder dessen physiologisch verträglichen Salze oder Ester in Kombination mit einem oder mehreren Wirkstoffen der Gruppe Meticlorpindol, Methyl Benzoquat, Amprolium, Beclotiamine oder Halofuginon oder dessen Salz in einer synergistisch wirksamen Menge.

Insbesondere kommen Zweierkombinationen in Betracht. Erfindungsgemäß könnnen auch Dreierkombinationen verwendet werden; von den letzten ist bevorzugt die Kombination von Narasin oder Salinomycin mit Meticlorpindol und Methyl Benzoquat.

Außerdem kann anstelle von Amprolium dessen Gemisch mit Ethopabate erfindungsgemäß eingesetzt werden.

Die erfindungsgemäß zu verwendenden Kombinationspartner für Salinomycin bzw. Narasin sind in der Veterinärmedizin seit längerer Zeit bekannt. Sie werden alle im Merck Index, 10. Aufl., veröff. durch Merck & Co., Inc. USA (1983) beschrieben: Meticlorpindol (3,5-Dichlor-2,6-dimethyl-4pyridinol) auf S. 341; Methyl Benzoquat (Nequinate; 3-Methoxycarbonyl-6-n-butyl-7-benzyloxy-4-oxochinolin auf S. 928; Ethopabate (4-Acetamido-2-ethoxy-benzoesäuremethylester) S. 544; Amprolium (1-[(4-Amino-2-propyl-5-pyrimidinyl)-methyl ]-2-methyl-piperidinium-chlorid) S. 613; Beclotiamine (3-[(4-Amino-2-methyl-5-pyrimidinyl)methyl ]-5-(2-chlorethyl)-4-methyl-thiazolium-chlorid) S. 144 und Halofuginon (7-Brom-6-Chlor-Febrifugine) aus S. 662. Neben Halofuginon werden erfindungsgemäß auch deren physiologisch verträgliche Salze wie das Hydrohalogenid, insbesondere Hydrobromid, Acetat, Lactat, Alkali- oder Erdalkalisalz, Salz der Acetursäure, s. DE-OS 2934069 erfaßt, sowie alle optischen Isomeren bzw. deren Gemische. Amprolium oder Beclotiamine können in Form ihrer Hydrochlorid-Additionssalze eingesetzt werden. Anstelle von Beclotiamine kann auch dessen entsprechendes Naphthalin-1,5-disulfonsäuresalz eingesetzt werden.

Die Kombination von Meticlorpindol und Methyl Benzoquat ist als Handelsprodukt ®Lerbek (Fa. ICI) bekannt. Die Kombination von Amprolium mit Ethopabate ist als Handelsprodukt ®Amprol Mix Super auf dem Markt (Merck, Sharp & Dohme).

Salinomycin oder Narasin können als freie Säure, als Salz, insbesondere als Alkali-(Na, K)-, Erdalkali-(Mg,Ca)- oder Ammoniumsalz, oder als Ester, insbesondere als $(C_1-C_4)$-Alkylester oder Benzylester eingesetzt werden; bevorzugt wird das Na-Salz verwendet. Die Produkte können in gereinigter Form oder als Mycel oder Rohprodukt eingesetzt werden.

Die erfindungsgemäßen Kombinationen zeigen synergistische Effekte bei der Bekämpfung der Coccidiose, insbesondere der Geflügelcoccidiose.

Bei der Massenhaltung von Geflügel auf engem Raum, z.B. Gelügelmast oder Geflügelaufzucht, besteht stets die potentielle Gefahr einer durch Eimeria-Spezies verursachten Coccidieninfektion, die ohne chemotherapeutische Bekämpfung zu ernsten wirtschaftlichen Verlusten führt. Coccidieninfektionen verursachen in der Regel Gewichtsdepression und blutige Kotausscheidungen, die als Folge von Läsionen in der Darmschleimhaut auftreten. Schwere Coccidieninfektionen führen beim Geflügel in der Regel auch zu hoher Mortalität.

Durch die Anwendung der erfindungsgemäßen Kombinationen ergeben sich im Vergleich zu den entsprechenden Einzelwirkstoffen erhebliche Vorteile, da geringere Mengen an coccidioziden Mitteln eingesetzt werden können. Hieraus resultieren eine Reduzierung der toxischen Nebenwirkungen der Präparate im Vergleich zur Applikation der Einzelwirkstoffe, eine erhöhte Wirtschaftlichkeit sowie Verringerung der Rückstände in den eßbaren Geweben des Geflügels.

In den erfindungsgemäßen Mitteln können die Gewichtsverhältnisse der Wirkstoffe in weiten Grenzen variieren. Sie liegen bei Kombinationen von Salinomycin bzw. Narasin

a) mit Meticlorpindol zwischen 5:1 bis 1:25, insbesondere zwischen 1,5:1 bis 1:5

b) mit Methyl Benzoquat zwischen 100:1 bis 1:3, insbesondere zwischen 60:1 bis 5:1

c) mit Halofuginon zwischen 160:1 bis 3:1, insbesondere zwischen 100:1 bis 8:1

d) mit einem Gemisch von Meticlorpindol und Methyl Benzoquat zwischen 20:1 bis 1:20, insbesondere 10:1 bis 1:5

e) mit Amprolium, zwischen 1:1 und 1:5

f) mit Beclotiamine, zwischen 1:1 und 1:5

g) mit einem Gemisch von Amprolium und Ethopabate, zwischen 1:1 und 1:5 (Jeweils Verhältnis Salinomycin oder Narasin zum Kombinationspartner) Das Verhältnis von Meticlorpindol zu Methyl Benzoquat kann im Falle d) zwischen 20:1 bis 7:1 variieren, während das Verhältnis Amprolium zu Ethopabate im Falle g) zwischen 30:1 und 10:1 variiert.

Die erfindungsgemäßen Kombinationen eignen sich ganz allgemein zum Schutz von Geflügel, d.h. zur Behandlung von landwirtschaftlichem Nutzgeflügel, wie Hühner, Truthühner, Enten oder Gänsen oder auch anderen Vögeln, wie z.B. Fasanen, Wachteln oder Perlhühnern; letztere Vogelarten werden neuerdings auch in Farmen zur wirtschaftlichen Nutzung gehalten und ebenso wie Hühner häufig und massiv von Coccidien befallen.

Zum erfolgreichen Schutz des Geflügels gegen Coccidiose können die erfindungsgemäßen Mittel zu jeder Zeit eingesetzt werden. Sie können insbesondere in Masthühnerbetrieben oder Aufzuchthäusern von Junghennen Anwendung finden, da dort aufgrund der dauernd im Kot ausgeschiedenen Dauerformen der Coccidien (Oocysten) ein hoher Infektionsdruck Für die Geflügelpopulation entsteht. Da unter diesen Bedingungen stets das Risiko eines Coccidioseausbruches besteht, sollten die erfindungsgemäßen coccidioziden Mittel beim Geflügel kontinuierlich und vor Ausbruch einer Coccidiose eingesetzt werden. Die erfindinngsgemäßen Mittel können aber auch während kurzer Zeitintervalle, d.h. weniger Tage verabreicht werden.

Der Einsatz der erfindungsgemäßen Mittel und Methoden zur Bekämpfung von Coccidiose wird in üblicher Weise durchgeführt. Entsprechend der Lokalisation der Coccidien im Intestinaltrakt kommt in erster Linie eine orale Applikation infrage. Die erfindungsgemäßen Wirkstoffkombinationen können dabei mit Futtermitteln oder mit Trinkwasser gemischt sein.

Die Wirkstoffkonzentrationen der Kombinationen in den Futtermitteln oder im Trinkwasser können innerhalb bestimmter Grenzen variieren. Sie liegen im allgemeinen zwischen 5 und 300 ppm der Wirkstoffkombination bezogen auf das Futtermittel bzw. Trinkwasser.

Die besonders bevorzugten Konzentrationen für die Bekämpfung der Coccidiose sind im Falle des Futtermittels jeweils 15 bis 70 ppm insbesondere 25 bis 45 ppm, Salinomycin oder Narasin und

a) 15 bis 150 ppm, insbesondere 30 bis 125 ppm Meticlorpindol

b) 1 bis 30 ppm, insbesondere 1,25 bis 5 ppm Methyl Benzoquat

c) 0,5 bis 3 ppm, insbesondere 0,75 bis 1,5 ppm Halofuginon und

d) 5 bis 120 ppm, insbesondere 8 bis 30 ppm Meticlorpindol/Methyl Benzoquat

e) 100 bis 150 ppm Amprolium

f) 40 bis 125 ppm Beclotiamine

g) 30 bis 100 ppm Amprolium/Ethopabat.

Im Falle der Verwendung im Trinkwasser wird bevorzugt jeweils ca. die Hälfte der angegebenen Konzentrationen benutzt.

Alle erwähnten Konzentrationen, Verhältnisse, Teile, Mengen oder Prozentangaben sind auf Gewichtseinheiten bezogen.

Die Wirkstoffkonzentrationen der coccidioziden Mittel sind auf Futter- oder Trinkwasserzubereitungen ad libitum bezogen, d.h. zur freien Futter- oder Trinkwasseraufnahme während einer praxisüblichen Mast- oder Aufzuchtperiode. Aufgrund besonderer durch die Praxis bedingter Faktoren kann es sich aber ergeben, daß der Geflügelfachmann diese Anwendungskonzentrationen nach oben anpassen muß, falls das Geflügel mit verschiedenen Futter- oder Wasservorräten versorgt werden muß. Dann enthält aber nur ein Teil der Futter-oder Wasservorräte die erfindungsgemäßen Mittel.

Als Träger für die erfindungsgemäßen synergistisch wirksamen coccidioziden Mittel eignen sich alle in der Geflügelindustrie üblichen Futterformulierungen. Die im folgenden angegebenen Formulierungen für Geflügelfutter sind Beispiele für praxisübliche Formulierungen. Darüber hinaus lassen sich aber auch andere Futtermittel auf der Basis von irgendwelchen Getreidekörnern verwenden, die Vitaminkonzentrate, Mineralkonzentrate oder sonstige Wirkstoffe und Futterzusätze in beliebiger Konzentration enthalten. Sowohl herkömmliche trockene, mehlige oder pelletierte Futtermittel als auch flüssige Suspensionsfutter unter Einschluß von Futtermitteln, wie Destillationsschlempen und Milchnebenprodukten, können bei den erfindungsgemäßen Mitteln verwendet werden.

Für die Herstellung des erfindungsgemäßen Geflügelfutters setzt man gewöhnlich zuerst ein konzentriertes Vorgemisch an, das die synergistisch wirksamen coccidioziden Mittel in hoher Konzentration, beispielsweise von 0,2 bis 75 %, enthält. Zu diesem Zweck werden die coccidioziden Mittel mit physiologisch unbedenklichen Trägern, wie Propylenglykolen, Polyethylenglykolen, inerten Ölen, wie Pflanzenölen, hochraffinierten Mineralölen, Äthanol, Wasser, wässrigen Alkoholen, entweder dispergiert oder in inerte Trägerstoffe eingemischt, wie Vermikulit, Diatomeenerde, Attapulgit, Kalziumkarbonat, Bolus alba. Ebenso eignen sich hierfür organische Trägermaterialien, wie Weizenkleie, Maisschrot, Sojabohnenmehl, Luzernemehl, Reishülsen oder gemahlene Maiskolben und beliebig andere organische Trägerstoffe aus pflanzlichen Produkten.

Die erfindungsgemäßen synergistisch wirksamen Mittel lassen sich ferner auch zusammen mit dem Trinkwasser an Geflügel verabreichen. Ihre Einarbeitung in das Trinkwasser wird durch Zusatz einer wasserlöslichen oder in Wasser suspendierbaren Form der jeweiligen Mittel zu dem Trinkwasser in geeigneter Menge erreicht. Die Herstellung solcher Zubereitungen erfolgt im allgemeinen durch Auswahl einer wasserlöslichen Form der Mittel. Sofern diese nicht gewünscht oder nicht herstellbar sind, können auch wasserunlösliche Formen, beispielsweise Suspensionen Verwendung finden. Zur Herstellung der Zubereitungen verwendet man physiologisch unbedenkliche Hilfsmitel, durch die die erfindungsgemäßen coccidioziden Mittel in Wasser über längere Zeit in Suspensionen gehalten werden. Hilfsmittel, die sich hierfür eignen sind Quellmittel wie Alginate, Gelatine, Carboxymethylcellulose oder Polyvinylpyrrolidon. Die erfindungsgemäßen Mittel können aber auch mit verschiedenen oberflächenaktiven Verbindungen suspendiert werden, wie beispielsweise mit Hilfe von Lecithin, Napthalinsulfonaten, Alkylbenzolsulfonaten, alkylphenol-Polyethylenoxid-Addukten oder Polyoxyethylensorbitanestern. Üblicherweise stellt man zunächst eine konzentrierte Suspension oder auch eine trockene Formulierung aus den erfindungsgemäßen coccidioziden Mitteln und den Suspendierungsmitteln in bestimmten Mischungsverhältnissen her und verdünnt sie dann mit dem Trinkwasser auf die gewünschte Anwendungskonzentration, oder die Vormischungen werden in geeigneten Konzentration mit den in der Praxis üblichen Futtermitteln gemischt. Das so medikierte Trinkwasser bzw. Futter wird dann dem Geflügel entweder zunächst ad libitum oder eine bestimmte Zeit angeboten.

Das erfindungsgemäßen Behandlungsverfahren läßt sich auch auf andere Verfahren zur Behandlung und Fütterung von Geflügel erweitern. So können beispielsweise die erfindungsgemäßen Zubereitungen mit anderen Wirkstoffen kombiniert werden, wie z.B. mit wachstumsfördernden Mitteln oder Parasitika, die einerseits synthetische Mittel oder andererseits Fermentationsprodukte im weitesten Sinn darstellen.

Die Erfindung ist zwar im besonderen Maße auf den Schutz des Geflügels gegenüber Coccidieninfektionen gerichtet, ist jedoch auch sinngemäß bei anderen Haus- und Nutztieren, wie beispielsweise anderen Vögeln, Kaninchen, Schweinen und Wiederkäuern anzuwenden.

Gegenstand der Erfindung ist daher auch ein Verfahren zur Behandlung der Geflügelcoccidiose, das darin besteht, daß man obengenannte wirksame Mittel oral an Geflügel ad libitum verabreicht, und zwar entweder als Futtermittel oder mittels Trinkwasser.

Die Erfindung wird durch nachfolgende Beispiele erläutert.

A. Beispiele für Tierfutter-Zusammensetzungen

Die folgenden Beispiele beziehen sich auf Tierfutterzusammensetzungen, welche zur Applikation der erfindungsgemäßen Wirkstoffkombination benutzt werden können.

I. Mastfutter für Broiler

<u>Zusammensetzung in Gew.-%</u>

| | |
|---|---|
| Fischmehl (60 - 65 %) | 6,0 |
| Futterhefe | 2,0 |
| Rindertalg | 4,7 |
| Sojaschrot (44 %) | 24,0 |
| Luzernegrünmehl | 1,0 |
| Mais | 44,89 |
| Weizen | 6,35 |
| Weizennachmehl | 8,5 |
| Phosphors. Futterkalk | 1,4 |
| Kohlens. Futterkalk | 0,96 |
| Spurenelementvormischung* | 0,04 |
| Viehsalz | 0,09 |
| Methionin DL | 0,07 |
| | 100,0 |

a) <u>pro kg Futter werden zugesetzt:</u>

| | | |
|---|---|---|
| Vitamin A | i.E. | 12.000 |
| $D_3$ | i.E. | 1.500 |
| E | mg | 18 |
| $B_1$ | mg | 1,5 |
| $B_2$ | mg | 6 |
| Pantothensäure | mg | 9 |
| Nicotinsäure | mg | 24 |
| Vitamin $B_6$ | mg | 4,5 |
| $B_{12}$ | mcg | 24 |
| $K_3$ | mg | 3 |
| Cholinchlorid | mg | 1.300 |

*b) <u>In 1,0 kg Futter enthalten</u>

| | | |
|---|---|---|
| Mn | mg | 106 |
| Zn | mg | 71 |
| Fe | mg | 44 |
| Cu | mg | 3,56 |
| I | mg | 0,4 |
| Co | mg | 0,16 |

II. Aufzuchtfutter für Küken (0. - 8. Woche)

<u>Zusammensetzung in Gew.-%</u>

| | |
|---|---|
| Fischmehl (60 - 65 %) | 5,0 |
| Luzernegrünmehl | 6,0 |
| Sojaschrot (44 %) | 13,0 |
| Futterhefe | 2,8 |
| Rindertalg | 2,0 |
| Gerste | 6,0 |
| Hafer | 6,0 |
| Mais | 37,0 |
| Weizen | 13,0 |
| Weizenkleie | 7,3 |
| Kohlens. Futterkalk | 1,29 |
| (R) Hostaphos | 0,57 |
| Spurenelementvormischung * | 0,04 |
| | 100,0 |

* s. Beispiel I b); ferner werden , wie in Beispiel I a) beschrieben, Vitamine zugesetzt.

III. Aufzuchtfutter für Küken (9. - 20. Woche)

Zusammensetzung in Gew.-%

| | |
|---|---|
| Luzernegrünmehl | 6,0 |
| Sojaschrot (44 %) | 10,24 |
| Futterhefe | 1,8 |
| Rindertalg | 2,0 |
| Gerste | 8,0 |
| Hafer | 6,0 |
| Mais | 40,0 |
| Weizen | 15,0 |
| Weizenkleie | 8,3 |
| Kohlens. Futterkalk | 1,53 |
| (R)Hostaphos | 1,02 |
| Spurenelementvormischung * | 0,04 |
| Methionin DL | 0,07 |
| | 100,0 |

* s. Beispiel I b); ferner werden Vitamine zugesetzt, s. Beispiel I a)

IV. Aufzuchtfutter für Küken (ab 21. Woche)

Zusammensetzung in Gew.-%

| | |
|---|---|
| Fischmehl (60 - 65 %) | 1,5 |
| Sojaschrot (44 %) | 19,31 |
| Rindertalg | 1,0 |
| Hafer | 6,7 |
| Mais | 40,0 |
| Weizen | 18,0 |
| Weizennachmehl | 3,0 |
| Methionin DL | 0,07 |
| Futterpaprika | 0,3 |

7

Kohlens. Futterkalk          8,16

(R)Hostaphos          1,91

Spurenelementvormischung *    0,05

                     100,0

Ferner werden Vitamine, wie in Beispiel I a) beschrieben zugegeben.

V. <u>Futter für Puten</u> (Zusammensetzung in Gew.-%)

|  | Putenstarter-futter 0.-8. Woche | Putenmast-futter I 9.-12. W. | Putenmast-futter II 13.-16. W. |
|---|---|---|---|
| Fischmehl | 9,0 | 5,0 | 2,0 |
| Futterhefe | 4,5 | 2,91 | 4,0 |
| Fett |  | 3,1 | 6,7 |
| Sojaschrot | 26,0 | 23,9 | 23,87 |
| Luzernegrünmehl | 4,6 | 2,0 | 0,95 |
| Gerste | 5,3 | 4,5 | 5,0 |
| Hafer |  | 1,0 | 4,45 |
| Mais | 36,04 | 40,02 | 39,90 |
| Weizen | 5,3 | 6,0 | 5,0 |
| Weizennachmehl | 5,95 | 4,0 | 3,8 |
| Weizenkleie |  | 4,0 |  |
| Phosphors. Futterkalk | 1,58 | 1,30 | 2,71 |
| Kohlens. Futterkalk | 1,35 | 1,29 | 0,96 |
| Spurenelementvormischung * | 0,04 | 0,04 | 0,04 |
| Viehsalz | 0,24 | 0,84 | 0,44 |
| Methionin | 0,1 | 0,1 | 0,18 |
|  | 100,0 | 100,0 | 100,0 |

<u>Vitaminmischung pro kg Futter:</u>

| | | | | |
|---|---|---|---|---|
| Vitamin A | i.E. | 12.000 | 8.000 | 8.000 |
| $D_3$ | i.E. | 1.500 | 1.000 | 1.000 |
| E | mg | 18 | 12 | 12 |
| $B_1$ | mg | 1,5 | 1 | 1 |
| $B_2$ | mg | 6,0 | 4 | 4 |

| | | | | |
|---|---|---|---|---|
| Pantothensäure | mg | 9,0 | 6 | 6 |
| Nicotinsäure | mg | 24,0 | 16 | 16 |
| Vitamin $B_6$ | mg | 4,5 | 3 | 3 |
| $B_{12}$ | mcg | 24,0 | 16 | 16 |
| $K_3$ | mg | 3,0 | 2 | 2 |
| Cholinchlorid | mg | 1.300 | 1.300 | 1.300 |

\* in einem 1 kg Futter enthalten: 106 mg Mn; 71 mg Zn; 44 mg Fe; 3,56 mg Cu; 0,4 mg I; 0,16 mg Co.

B. Biologische Beispiele

Der coccidiostatische Effekt der vorliegenden Mittel wurde an mit Coccidien infizierten Hühnchen untersucht. Die im folgenden aufgeführten experimentellen Untersuchungen zeigen die Wirksamkeit der verschiedenen erfindungsgemäßen Kombinationen anhand einiger Beispiele. Das Salinomycin wird hierbei in Form eines Natriumsalzes des nicht aufgetrennten und durch Fermentation hergestellten Mycels eingesetzt.

Zur Bestimmung und Bewertung des coccidiostatischen Effektes der erfindungsgemäßen Mittel wurden bei allen folgenden beschriebenen experimentellen Untersuchungen sogenannte Infektionskontrollen (unbehandelte, infizierte Tiere) und O-Kontrollen (unbehandelte, nicht infizierte Tiere) verwandt. Die hierfür verwendeten Tiere beiderlei Geschlechts (LSL Hühnchen, Fa. Lohmann, Wallau, BRD) wurden randomisiert und jeweils Gruppen von 16 Tieren zusammengestellt. Nach der gleichen Methode wurden die mit den erfindungsgemäßen Mitteln behandelten und infizierten Gruppen zusammengestellt. Die Tiere, ausgenommen diejenigen der O-Kontrolle, wurden mit einem virulenten Eimeria tenella-Stamm infiziert, der in 8 Tage alten Hühnchen zu starken (reproduzierbaren) Läsionen der Blinddärme führt. Die coccidioziden Mittel wurden mit dem Futter für Geflügel in ppm-Mengen vermischt; die jeweiligen Konzentrationen sind aus den folgenden Tabellen 1a bis 1j zu ersehen.

Der Grad der durch die Infektion hervorgerufenen pathologisch-anatomischen Veränderungen der Darmschleimhaut in den Blinddärmen wird üblicherweise (Experimental Parasitology Vol. 28, 1970; oder Long, P.L.: The Biology of the Coccidia, 1982, Univ. Park Press) in Form von Schädigungswerten (= Läsionswerten) im folgenden "lesion scores" genannt, (Skala von 0 bis 4) ausgedrückt. Am Ende der Untersuchungen wurden die Tiere 5 Tage nach der Infektion getötet und alle auf typische durch Coccidiose verursachte pathologisch-anatomischen Veränderungen untersucht.

Beschreibung und Bewertung der Schädigung (lesion scores): 0 bis 4

0 = Tiere, bei denen keine Läsionen im Intestinaltrakt nachweisbar sind

1 = Tiere mit wenigen, umschriebenen kleinen Läsionen (Petechien) in den Blinddärmen

2 = Tiere mit mehreren, umschriebenen und etwas größeren Läsionen (Petechien) als unter 1 beschrieben

3 = Tiere mit zahlreichen und relativ großflächigen, blutigen Läsionen, die teilweise konfluieren.

4 = Tiere mit großflächigen, blutigen Läsionen, die die gesamte Darmschleimhaut der Blinddärme und auch angrenzende Darmabschnitte (Hüftdarm = Ileum bzw. Mastdarm) erfassen. Es bietet sich das Bild einer großflächigen hämorrhagischen Enteritis schwersten Grades, die in der Regel zum Tod des Tieres führt.

Bei den unter 1 bis 4 angegebenen Schädigungen wird in zunehmendem Maß dünnflüssiger und blutiger Kot abgesetzt. Die Gewichtsdepression infolge Nahrungsverweigerung korreliert ebenso mit der Zunahme der "lesion scores".

Aus den erwähnten Beobachtungen wird die Relevanz der lesion scores für die Beurteilung der coccidioziden Wirkung der erfindungsgemäßen Mittel deutlich.

Die lesion scores werden einmal als Einzelwerte pro Tier innerhalb einer Gruppe, zum anderen als Mittelwerte der entsprechenden Tiergruppe in den Tabelle 1a - 1j aufgeführt.

Behandlung und Infektionen

In allen Versuchen wurden jeweils 16 eine Woche alte LSL-Hühnchen pro Gruppe unter konstanten Raumbedingungen in Drahtkäfigen zu jeweils 4 Tieren pro Drahtkäfig gehalten. Das mit den erfindungsgemäßen Mitteln medikierte Futter wurde vom Tage D-1 (ein Tag vor der Infektion) bis zum Tage D + 5 (5 Tage nach der Infektion) ad libitum angeboten. Die Kontrollgruppen (0-Gruppe bzw. Infektionskontrolle) erhielten nicht medikiertes Futter. Die jeweiligen Tiergruppen wurden somit 7 Tage mit dem gleichen Futter gefüttert. Am Tag der Infektion (= DO) wurden je Tier 200.000 sporulierte Oocysten obengenannten E. tenella-Stammes per Schlundsonde appliziert.

Nach Andauung der Oocysten im Dünndarm werden aus diesen Dauerformen die infektiösen Sporozoiten freigesetzt, die anschließend die Epithelzellen der Darmschleimhaut befallen und sich dort massenhaft durch Formenänderung vermehren. Die aus den Sporozoiten sich entwickelnden zahlreichen Schizonten zerstören durch wiederholte Teilungsvorgänge das Darmepithel. Der Höhepunkt des zerstörenden Effektes der Schizonten auf das Darmepithel Ist 5 Tage nach der Infektion erreicht. Deshalb wurden zu diesem Zeitpunkt in der oben beschriebenen Weise die "lesion scores" ermittelt.

Der synergistische Effekt der erfindungsgemäßen Mittel (Kombinationen) wird im folgenden in den Tabellen 1a - 1j dargestellt, und zwar einmal der Effekt der Einzelwirkstoffe in der praxisüblichen Anwendungskonzentration und zum anderen der aus verschiedenen Kombinationen der Einzelwirkstoffe resultierende überadditive oder synergistische Effekt. Die "lesion scores" der Infektionskontrollen bzw. der O-Kontrollen (nicht infizierte Tiere) dienen der Abgleichung mit den jeweiligen medikierten und infizierten Tiergruppen.

10

## Tabelle 1a

### Kombinationen Salinomycin mit Meticlorpindol

| Behandlung (medikiertes Futter) | Konzentration ppm | Schädigung Einzelwert pro Tier | (lesion scores) Summe | Mittelwert pro Gruppe |
|---|---|---|---|---|
| keine (Infektionskontrolle) | 0 | 3 3 3 4 4 4 4 4<br>4 4 4 4 4 4 4 4 | 61 | 3,8 |
| keine (nicht infiz. Kontrolle) | 0 | 0 0 0 0 0 0 0 0<br>0 0 0 0 0 0 0 0 | 0 | 0 |
| Salinomycin | 60 | 0 0 0 0 0 0 0 0<br>0 0 0 0 0 0 0 0 | 2 | 0,13 |
|  | 30 | 3 3 3 3 3 3 3 4<br>4 4 4 4 4 4 4 4 | 57 | 3,6 |
| Meticlorpindol | 125 | 0 0 0 0 0 0 0 0<br>0 0 0 0 0 1 1 1 | 3 | 0,18 |
|  | 62,5 | 1 1 1 2 2 2 2 2<br>2 2 2 2 2 2 3 3 | 31 | 1,9 |
|  | 30 | 3 3 3 3 3 4 4 4<br>4 4 4 4 4 4 4 4 | 59 | 3,7 |
| Salinomycin + Meticlorpindol | 30+62,5 | 0 0 0 0 0 0 0 0<br>0 0 0 0 0 0 0 0 | 0 | 0 |
|  | 30+30 | 0 0 0 0 0 0 0 0<br>0 0 0 0 0 0 0 1 | 1 | 0,06 |

EP 0 246 532 B1

Tabelle 1b  Kombinationen Salinomycin mit Methyl Benzoate

| Behandlung (medikiertes Futter) | Konzentration ppm | Schädigung (lesion scores) Einzelwert pro Tier | | Summe | Mittelwert pro Gruppe |
|---|---|---|---|---|---|
| keine (Infektionskontrolle) | 0 | 3 3 3 3 3 4 4 4<br>4 4 4 4 4 4 4 4 | | 59 | 3,7 |
| keine (nicht infiz. Kontrolle) | 0 | 0 0 0 0 0 0 0 0<br>0 0 0 0 0 0 0 0 | | 0 | 0 |
| Salinomycin | 60 | 0 0 0 0 0 0 0 0<br>0 0 0 0 0 0 0 0 | | 2 | 0,13 |
| | 30 | 2 3 3 3 3 3 3 3<br>4 4 4 4 4 4 4 4 | | 55 | 3,4 |
| | 15 | 3 3 3 3 3 3 3 4<br>4 4 4 4 4 4 4 4 | | 57 | 3,6 |
| Methyl Benzoquate | 15 | 0 0 0 0 0 0 0 0<br>0 0 0 0 0 1 1 1 | | 3 | 0,19 |
| | 5 | 0 0 1 1 2 2 2 2<br>2 2 2 2 2 3 3 3 | | 29 | 1,8 |
| | 2,5 | 0 0 2 2 3 3 4 4<br>4 4 4 4 4 4 4 4 | | 50 | 3,1 |
| | 1,25 | 3 3 3 3 4 4 4 4<br>4 4 4 4 4 4 4 4 | | 60 | 3,8 |
| Salinomycin + Methyl Benzoquate | 30+5 | 0 0 0 0 0 0 0 0<br>0 0 0 0 0 0 0 1 | | 1 | 0,06 |
| | 30+2,5 | 0 0 0 0 0 0 0 0<br>0 0 0 2 2 2 3 3 | | 12 | 0,8 |
| | 30+1,25 | 0 0 0 0 2 2 2 3<br>3 3 3 3 4 4 4 4 | | 35 | 2,2 |
| | 15+2,5 | 0 0 0 0 0 0 0 0<br>2 2 2 3 3 4 4 4 | | 24 | 1,5 |
| | 15+1,25 | 0 0 0 0 0 2 2 2<br>2 3 3 4 4 4 4 4 | | 34 | 2,1 |

EP 0 246 532 B1

EP 0 246 532 B1

## Tabelle 1c

### Kombinationen Salinomycin mit Meticlorpindol und Methyl Benzoquat

| Behandlung (medikiertes Futter) | Konzentration ppm | Schädigung Einzelwert pro Tier | (lesion scores) Summe | Mittelwert pro Gruppe |
|---|---|---|---|---|
| keine (Infektionskontrolle) | 0 | 3 3 3 3 3 4 4 4 <br> 4 4 4 4 4 4 4 4 | 59 | 3,7 |
| keine (nicht infiz. Kontrolle) | 0 | 0 0 0 0 0 0 0 0 <br> 0 0 0 0 0 0 0 0 | 0 | 0 |
| Salinomycin | 30 | 2 3 3 3 3 3 3 3 <br> 4 4 4 4 4 4 4 4 | 55 | 3,4 |
| Kombination Meticlorpindol (100 Teile) + | 27,5 | 1 1 1 1 2 2 2 2 <br> 2 2 2 2 2 3 3 3 | 31 | 1,9 |
| Methyl Benzoquat (9,35 Teile) | 9,5 | 2 2 3 3 3 3 3 3 <br> 3 3 3 4 4 4 4 4 | 51 | 3,2 |
| Salinomycin + (Meticlorpindol/ | 30+27,5 | 0 0 0 0 0 0 0 0 <br> 0 0 0 0 0 0 0 0 | 0 | 0 |
| Methyl Benzoquat | 30+9,5 | 0 0 0 0 0 0 0 0 <br> 0 0 0 0 1 2 2 2 | 7 | 0,43 |

## Tabelle 1d

### Kombinationen Salinomycin mit Halofuginon

| Behandlung (medikiertes Futter) | Konzentration ppm | Schädigung Einzelwert pro Tier | (lesion scores) Summe | Mittelwert pro Gruppe |
|---|---|---|---|---|
| keine (Infektionskontrolle) | 0 | 2 3 3 4 4 4 4 4<br>4 4 4 4 4 4 4 4 | 60 | 3,8 |
| keine (nicht infiz. Kontrolle) | 0 | 0 0 0 0 0 0 0 0<br>0 0 0 0 0 0 0 0 | 0 | 0 |
| Salinomycin | 30 | 2 2 2 3 3 3 3 3<br>3 4 4 4 4 4 4 4 | 52 | 3,3 |
| Halofuginon | 1,5 | 1 1 2 2 2 2 2 2<br>2 2 2 3 3 3 3 3 | 34 | 2,1 |
| | 0,75 | 2 2 2 2 3 3 3 3<br>3 4 4 4 4 4 4 4 | 51 | 3,1 |

EP 0 246 532 B1

**Tabelle 1** *e*  Kombinationen von Salinomycin mit Amprolium und Ethopabate

| Behandlung (medikiertes Futter) | Konzentration ppm | Schädigung (lesion scores) Einzelwert pro Tier | Summe | Mittelwert pro Gruppe |
|---|---|---|---|---|
| keine Infektionskontrolle | 0 | 3 3 3 3 3 3 3 3<br>4 4 4 4 4 4 4 4 | 56 | 3.5 |
| keine Infektionskontrolle | 0 | 0 0 0 0 0 0 0 0<br>0 0 0 0 0 0 0 0 | 0 | 0 |
| Salinomycin | 60 | 0 0 0 0 0 0 0 0<br>0 0 0 0 0 0 0 1 | 1 | 0.1 |
| | 30 | 0 1 1 1 1 2 2 3<br>3 3 3 3 3 3 3 4 | 36 | 2.3 |
| Amprolium + Ethopabate (25 Teile + 1,6 Teile) | 125 | 0 0 0 0 0 0 0 0<br>0 0 0 0 0 0 1 1 | 2 | 0.1 |
| | 62.5 | 0 0 0 0 0 0 0 0<br>0 0 0 1 1 1 1 1 | 5 | 0.3 |
| | 31.25 | 1 1 2 3 3 3 3 4<br>4 4 4 4 4 4 4 4 | 52 | 3.3 |
| Salinomycin + Amprolium+Ethopabate | 30 + 62.5 | 0 0 0 0 0 0 0 0<br>0 0 0 0 0 0 0 0 | 0 | 0 |
| | 30 + 31.25 | 0 0 0 0 0 0 0 0<br>0 0 1 1 1 1 1 3 | 8 | 0.5 |

**Tabelle 15**    Kombinationen von Narasin mit Meticlorpindol

| Behandlung (medikiertes Futter) | Konzentration ppm | Schädigung (lesion scores) Einzelwert pro Tier | | Summe | Mittelwert pro Gruppe |
|---|---|---|---|---|---|
| keine Infektionskontrolle | 0 | 3 3 3 3 3 3 3 3 | 4 4 4 4 4 4 4 4 | 56 | 3.5 |
| keine Infektionskontrolle | 0 | 0 0 0 0 0 0 0 0 | 0 0 0 0 0 0 0 0 | 0 | 0 |
| Narasin | 70 | 0 0 0 0 0 0 0 0 | 2 2 2 3 3 3 3 3 | 21 | 1.3 |
| | 35 | 0 1 3 3 3 3 3 3 | 3 3 3 4 4 4 4 4 | 48 | 3.0 |
| Meticlorpindol | 125 | 0 0 0 0 0 0 0 0 | 0 0 0 0 0 0 0 0 | 0 | 0 |
| | 60 | 0 0 0 0 0 0 0 0 | 0 0 0 1 1 1 1 1 | 5 | 0.3 |
| | 30 | 1 3 3 3 3 3 3 3 | 4 4 4 4 4 4 4 4 | 54 | 3.4 |
| Narasin + Meticlorpindol | 35 + 60 | 0 0 0 0 0 0 0 0 | 0 0 0 0 0 0 0 0 | 0 | 0 |
| | 35 + 30 | 0 0 0 0 0 0 0 0 | 0 0 0 0 1 3 3 3 | 10 | 0.6 |

EP 0 246 532 B1

Tabelle 1g        Kombinationen von Narasin mit methyl Benzoquat

| Behandlung (medikiertes Futter) | Konzentration ppm | Schädigung (lesion scores) Einzelwert pro Tier | Summe | Mittelwert pro Gruppe |
|---|---|---|---|---|
| keine Infektionskontrolle | 0 | 3 3 3 3 3 3 3 3<br>4 4 4 4 4 4 4 4 | 56 | 3.5 |
| keine Infektionskontrolle | 0 | 0 0 0 0 0 0 0 0<br>0 0 0 0 0 0 0 0 | 0 | 0 |
| Narasin | 70 | 0 0 0 0 0 0 0 0<br>2 2 2 3 3 3 3 3 | 21 | 1.3 |
|  | 35 | 0 1 3 3 3 3 3 3<br>3 3 3 4 4 4 4 4 | 48 | 3.0 |
|  | 17.5 | 1 2 2 3 3 3 3 3<br>3 3 4 4 4 4 4 4 | 50 | 3.1 |
| Methyl Benzoquat | 10 | 0 0 0 0 0 0 0 0<br>0 0 0 0 0 0 0 0 | 0 | 0 |
|  | 2.5 | 0 0 0 0 0 0 0 1<br>3 3 3 4 4 4 4 | 29 | 1,8 |
| Narasin + Methyl Benzoquat | 35 + 2.5 | 0 0 0 0 0 0 0 0<br>0 0 0 0 0 0 0 | 0 | 0 |
|  | 17.5 + 2.5 | 0 0 0 0 0 0 0 0<br>0 0 0 0 0 1 1 3 | 5 | 0.3 |

EP 0 246 532 B1

EP 0 246 532 B1

**Tabelle 1h**      Kombinationen von Narasin mit Meticlorpindol und *Methyl Benzoquat*

| Behandlung (medikiertes Futter) | Konzentration ppm | Schädigung (lesion scores) Einzelwert pro Tier | Summe | Mittelwert pro Gruppe |
|---|---|---|---|---|
| keine Infektionskontrolle | 0 | 3 3 3 3 3 3 3 3<br>4 4 4 4 4 4 4 4 | 56 | 3.5 |
| keine Infektionskontrolle | 0 | 0 0 0 0 0 0 0 0<br>0 0 0 0 0 0 0 0 | 0 | 0 |
| Narasin | 70 | 0 0 0 0 0 0 0 0<br>2 2 2 3 3 3 3 3 | 21 | 1.3 |
| Narasin | 35 | 0 1 3 3 3 3 3 3<br>3 3 3 4 4 4 4 4 | 48 | 3.0 |
| Meticlorpindol/ Methyl Benzoquat (100 Teile + 9.35 Teile) | 108.35 | 0 0 0 0 0 0 0 0<br>0 0 0 0 0 0 0 0 | 0 | 0 |
| Meticlorpindol/ Methyl Benzoquat (100 Teile + 9.35 Teile) | 9.5 | 0 0 0 0 0 1 1 1<br>1 1 2 3 3 3 3 3 | 22 | 1.4 |
| Meticlorpindol/ Methyl Benzoquat (100 Teile + 9.35 Teile) | 6.75 | 0 1 1 1 1 3 3 4<br>4 4 4 4 4 4 4 4 | 46 | 2.9 |
| Narasin + Meticlorpindol/ Methyl Benzoquat | 35 + 9.5 | 0 0 0 0 0 0 0 0<br>0 0 0 0 0 0 0 0 | 0 | 0 |
| Narasin + Meticlorpindol/ Methyl Benzoquat | 35 + 6.75 | 0 0 0 0 0 0 0 0<br>0 1 1 1 1 3 3 3 | 13 | 0.8 |

**Tabelle 1i.**  Kombinationen von Narasin mit Halofuginon

| Behandlung (medikiertes Futter) | Konzentration ppm | Schädigung (lesion scores) Einzelwert pro Tier | | Summe | Mittelwert pro Gruppe |
|---|---|---|---|---|---|
| keine Infektionskontrolle | 0 | 3 3 3 3 3 3 3 4 | 4 4 4 4 4 4 4 4 | 57 | 3.6 |
| keine Infektionskontrolle | 0 | 0 0 0 0 0 0 0 0 | 0 0 0 0 0 0 0 0 | 0 | 0 |
| Narasin | 70 | 0 0 0 0 0 0 0 0 | 0 0 1 1 2 4 4 4 | 16 | 1 |
| | 35 | 1 1 1 1 1 1 2 3 | 3 4 4 4 4 4 4 4 | 42 | 2.6 |
| Halofuginon | 3 | 0 0 0 0 0 0 0 0 | 0 0 0 0 0 0 0 0 | 0 | 0 |
| | 1.5 | 0 0 0 0 0 0 0 0 | 1 1 1 1 3 3 3 3 | 16 | 1.0 |
| | 0.75 | 0 1 3 3 3 3 3 3 | 3 3 4 4 4 4 4 4 | 49 | 3.1 |
| Narasin + Halofuginon | 35 + 1.5 | 0 0 0 0 0 0 0 0 | 0 0 0 0 0 0 1 1 | 2 | 0.1 |
| | 35 + 0.75 | 0 0 0 0 0 0 0 0 | 0 0 0 1 2 3 3 3 | 12 | 0.8 |

EP 0 246 532 B1

**Tabelle 1:**  Kombinationen von Narasin mit *Amprolium und Ethopabate*

| Behandlung (mediklertes Futter) | Konzentration ppm | Schädigung (lesion scores) Einzelwert pro Tier | Summe | Mittelwert pro Gruppe |
|---|---|---|---|---|
| keine Infektionskontrolle | 0 | 3 3 3 3 3 3 3 3<br>4 4 4 4 4 4 4 4 | 56 | 3.5 |
| keine Infektionskontrolle | 0 | 0 0 0 0 0 0 0 0<br>0 0 0 0 0 0 0 0 | 0 | 0 |
| Narasin | 70 | 0 0 0 0 0 0 0 0<br>2 2 2 3 3 3 3 3 | 21 | 1.3 |
| Narasin | 35 | 0 1 3 3 3 3 3 3<br>3 3 3 4 4 4 4 4 | 48 | 3.0 |
| Amprolium + Ethopabate (100 Teile + 6.4 Teile) | 125 | 0 0 0 0 0 0 0 0<br>0 0 0 0 0 0 1 1 | 2 | 0.1 |
| | 62.5 | 0 0 0 0 0 0 0 0<br>0 0 0 1 1 1 1 1 | 5 | 0.3 |
| | 31.25 | 1 1 2 3 3 3 3 4<br>4 4 4 4 4 4 4 4 | 52 | 3.3 |
| Narasin + Amprolium+Ethopabate | 35 + 62.5 | 0 0 0 0 0 0 0 0<br>0 0 0 0 0 0 0 0 | 0 | 0 |
| | 35 + 31.25 | 0 0 0 0 0 0 0 0<br>0 0 0 0 0 0 1 1 | 2 | 0.1 |

**Patentansprüche**

1. Coccidiozide Mittel, gekennzeichnet durch einen Gehalt an einem Polyetherantibiotikum aus der Gruppe Salinomycin oder Narasin oder deren physiologisch verträglichen Salze oder Ester in Kombination mit einem oder mehreren Wirkstoffen der Gruppe Meticlorpindol, Methyl Benzoquat, Amprolium, Beclotiamine oder Halofuginon oder dessen Salz in einer synergistisch wirksamen Menge.

2. Mittel gemäß Anspruch 1, dadurch gekennzeichnet, daß es Salinomycin oder Narasin in Form seiner physiologisch verträglichen Salze oder Ester enthält.

3. Mittel nach Anspruch 2, dadurch gekennzeichnet, daß es als Salze Alkali- und Erdalkalisalze enthält.

4. Mittel nach Ansprüchen 2 oder 3, dadurch gekennzeichnet, daß es als Salze das Natrium-, Kalium-, Ammoniumsalz, das Magnesium- oder Calciumsalz enthält.

5. Mittel nach einem oder mehreren der Ansprüche 1 bis 4, dadurch gekennzeichnet, daß es Salinomycin oder Narasin als Na-Salz enthält.

6. Mittel nach Anspruch 1, dadurch gekennzeichnet, daß es Salinomycin oder Narasin als Mycel oder Rohprodukt enthält.

7. Mittel gemäß einem oder mehreren der Ansprüche 1 bis 6, dadurch gekennzeichnet, daß es Salinomycin oder Narasin in Kombination mit Meticlorpindol und Methyl Benzoquat enthält.

8. Mittel gemäß einem oder mehreren der Ansprüche 1 bis 7, dadurch gekennzeichnet, daß es Salinomycin oder Narasin in Kombination mit Amprolium und Ethopabate enthält.

9. Mittel gemäß einem oder mehreren Ansprüchen 1 bis 8, dadurch gekennzeichnet, daß es zusätzlich Geflügelfutter oder Trinkwasser enthält.

10. Verfahren zur Herstellung der Mittel gemäß einem oder mehreren der Ansprüche 1 bis 9, dadurch gekennzeichnet, daß man die Wirkstoffkombinationen in geeigneter Form mit einem Futtermittel oder Trinkwasser oder galenischen Hilfs- und Zusatzstoffen mischt und gegebenenfalls in eine für die orale Verabreichung geeignete Applikationsform bringt.

**Claims**

1. A coccidiocidal agent which contains a polyether antibiotic selected from the group comprising salinomycin, narasin or their physiologically acceptable salt and ester in combination with one or more active compounds selected from the group comprising meticlorpindol, methyl benzoquate, amprolium, beclotiamine or halofuginone or its salt in a synergistically active amount.

2. An agent as claimed in claim 1, which contains salinomycin or narasin in the form of its physiologically acceptable salts or esters.

3. An agent as claimed in claim 2, which contains alkali metal or alkaline earth metal salts as the salts.

4. An agent as claimed in either of claims 2 or 3, which contains the sodium, potassium, ammonium, magnesium or calcium salt as the salts.

5. An agent as claimed in one or more of claims 1 to 4, which contains salinomycin or narasin as the Na salt.

6. An agent as claimed in claim 1, which contains salinomycin or narasin as the mycelium or crude product.

7. An agent as claimed in one or more of claims 1 to 6, which contains salinomycin or narasin in combination with meticlorpindol and methyl benzoquate.

8. An agent as claimed in one or more of claims 1 to 7, which contains salinomycin or narasin in combination with amprolium and ethopabate.

9. An agent as claimed in one or more of claims 1 to 8, which additionally contains poultry feed or drinking water.

10. A process for the preparation of an agent as claimed in one or more of claims 1 to 9, which comprises mixing the active compound combination in a suitable form with a feedstuff or drinking water or formulation auxiliaries or additives and, if appropriate, bringing the mixture into an administration form suitable for oral administration.

**Revendications**

1. Agents coccidiocides, caractérisés en ce qu'ils contiennent un antibiotique de type polyéther du groupe comprenant la salinomycine ou la narasine, ou ses sels ou esters physiologiquement tolérés, en combinaison avec un ou plusieurs principes actifs du groupe comprenant le méticlorpindol, le méthylbenzoquat, l'amprolium, la béclotiamine ou l'halofuginone, ou leurs sels, en une quantité à effet synergique.

2. Produit selon la revendication 1, caractérisé en ce qu'il contient de la salinomycine ou de la narasine sous forme de ses sels ou esters physiologiquement tolérés.

3. Produit selon la revendication 2, caractérisé en ce qu il contient comme sels des sels de métaux alcalins et alcalino-terreux.

4. Produit selon les revendications 2 ou 3, caractérisé en ce qu'il contient comme sels les sels de sodium, de potassium, d'ammonium, de magnésium ou de calcium.

5. Produit selon l'une ou plusieurs des revendications 1 à 4, caractérisé en ce qu'il contient de la salinomycine ou de la narasine sous forme de leurs sels de Na.

6. Produit selon la revendication 1, caractérisé en ce qu'il contient la salinomycine ou la narasine sous forme d'un mycélium ou d'un produit brut.

7. Produit selon l'une ou plusieurs des revendications 1 à 6, caractérisé en ce qu'il contient de la salinomycine ou de la narasine en combinaison avec du méticlorpindol et du méthylbenzoquat.

8. Produit selon l'une ou plusieurs des revendications 1 à 7, caractérisé en ce qu'il contient de la salinomycine ou de la narasine en combinaison avec de l'amprolium et de l'éthopabate.

9. Produit selon l'une ou plusieurs des revendications 1 à 8, caractérisé en ce qu'il contient en outre des aliments pour volailles ou de l'eau potable.

10. Procédé de préparation des produits selon l'une ou plusieurs des revendications 1 à 9, caractérisé en ce qu'on mélange les combinaisons de principes actifs, sous une forme appropriée, avec un produit alimentaire ou de l'eau potable ou des additifs et auxiliaires galéniques, et éventuellement on lui donne une forme pour administration, convenant à une administration orale.